Europäisches Patentamt

European Patent Office

Office européen des brevets

(11) Numéro de publication: **0 349 514**
A1

## (12) DEMANDE DE BREVET EUROPEEN

(21) Numéro de dépôt: **89870100.8**

(22) Date de dépôt: **22.06.89**

(51) Int. Cl.⁵: **A 61 K 31/71**
A 61 K 9/54

(30) Priorité: **01.07.88 BE 8800763**

(43) Date de publication de la demande:
**03.01.90 Bulletin 90/01**

(84) Etats contractants désignés:
**AT BE CH DE ES FR GB GR IT LI LU NL SE**

(71) Demandeur: **"PHARLYSE", Société Anonyme
Boulevard Royal, 2
Luxembourg (LU)**

(72) Inventeur: **Fossion,Jacques
18 rue du cours d'Eau
B-1428 Lillois (BE)**

**Baudier,Philippe
10 Avenue Blücher
B-1410 Waterloo (BE)**

**Deboeck,Arthur Marie c/o Galephar P.R.
P.B. Box 3468
Carolina P.R. 00628-3468 (US)**

(74) Mandataire: **Schmitz, Yvon et al
Bureau Gevers S.A. rue de Livourne 7 bte 1
B-1050 Bruxelles (BE)**

(54) Composition pharmaceutique d'érythromycine à libération prolongée, sa préparation et son utilisation.

(57) Composition pharmaceutique à base d'érythromycine du type à libération prolongée, comprenant un mélange d'éthylsuccinate d'érythromycine et de microgranules d'érythromycine base entérosolubles, la quantité d'éthylsuccinate d'érythromycine par rapport à la quantité totale d'érythromycine base présente dans la composition, ces quantités étant exprimées en pourcentage d'érythromycine base, étant de l'ordre de 20 à 80 %.

EP 0 349 514 A1

**Description**

**Composition pharmaceutique d'érythromycine à libération prolongée, sa préparation et son utilisation.**

L'érythromycine est un antibiotique de la famille des macrolides produit par streptomyces erythreus. Découverte en 1952, l'érythromycine reste un agent thérapeutique de valeur malgré le développement de nombreuses nouvelles classes d'antibiotiques. Dans de nombreux cas elle est préférée aux pénicillines pour lesquelles elle présente une alternative de choix en cas d'allergie à ces dernières.

L'érythromycine base est une substance cristalline, extrêmement amère, peu soluble dans l'eau. Elle peut être inactivée par les acides ce qui se traduit, lors du traitement, par une diminution d'absorption consécutive à sa mise en contact avec les sécrétions gastriques acides.

Afin de remédier à cet inconvénient, des modifications chimiques de la substance ainsi que des préparations galéniques ont été réalisées.

Les modifications chimiques visant à diminuer la sensibilité de l'érythromycine vis-à-vis des acides consistent en la préparation de sels tels que le stéarate, d'esters tels que l'éthylsuccinate et d'esters-sels tels que le sel de sulfate de lauryle de l'ester propionique ou estolate.

Les préparations galéniques consistent à isoler l'érythromycine base au moyen d'un film entérosoluble.

L'activité antibiotique de l'érythromycine est fonction de sa concentration plasmatique et il est actuellement admis que son activité se manifeste pour des concentrations plasmatiques égales ou supérieures à 0,5 - 1 microgramme d'érythromycine base par millilitre de plasma.

L'inconvénient majeur de l'érythromycine, dû à son temps de demi-vie plasmatique extrêmement court (t 1/2 = 1,6 h), est que les prises de médicaments lors du traitement, ne peuvent être espacées que de 6 heures au maximum afin de maintenir des concentrations plasmatiques efficaces.

Ces nombreuses prises journalières rendent le traitement extrêmement difficile à suivre pour le patient et sont dans de nombreux cas la cause première d'un échec thérapeutique.

Actuellement, les préparations galéniques, destinées à l'administration d'érythromycine, sont constituées de gélules, suspension ou comprimés d'estolate ou d'éthylsuccinate et de comprimés ou microgranules entérosolubles d'érythromycine base.

Toutes ces modifications tant chimiques que galéniques, apportées jusqu'à présent à l'érythromycine base n'ont pas permis de résoudre l'inconvénient des prises multiples quotidiennes. De plus, ces formes libèrent rapidement dans l'estomac une quantité importante d'érythromycine, ce qui peut induire des effets secondaires tels que nausées et vomissement, particulièrement chez les enfants.

L'objet de la présente invention est de remédier à ces inconvénients en diminuant le nombre de prises journalières de médicament tout en maintenant des taux plasmatiques thérapeutiques.

Il a été découvert, maintenant, qu'une forme galénique contenant en mélange de l'éthylsuccinate d'étythromycine avec des microgranules d'érythromycine base enrobés d'un film entérosoluble permettent l'obtention de taux plasmatiques efficaces tout en réduisant le nombre de prises de médicament. En effet, la nouvelle forme galénique d'érythromycine par administration orale, objet de la présente invention, permet une réduction du nombre de prises de médicament de 4 par jour à 2 par jour, c'est-à-dire de 50%.

Cette réduction permet, et ce pour la première fois, une posologie réaliste. En effet, il est connu que des administrations médicamenteuses espacées de 6 heures seulement sont extrêmement mal suivies par les patients car elles rendent obligatoire le réveil durant la nuit et sont donc à l'origine de fréquents échecs de traitements thérapeutiques.

Description détaillée de l'invention

La présente invention à pour objet de nouvelles compositions d'érythromycine caractérisées en ce qu'elles sont constituées d'un mélange d'éthylsuccinate d'érythromycine et de microgranules d'érythromycine base, enrobés d'une membrane entérosoluble.

Conformément à la présente invention, les nouvelles compositions peuvent contenir des proportions exprimées en pourcentage d'érythromycine base, pouvant être comprises entre 30 % et 70 % d'érythromycine sous forme d'éthylsuccinate par rapport à la quantité totale d'érythromycine présente dans la forme.

Selon une particularité de l'invention, les microgranules d'érythromycine base peuvent être mélangés à de l'éthylsuccinate d'érythromycine sous forme de poudre ou de granulé. Le mélange final peut être placé dans des capsules de gélatine dure, des sachets ou comprimés ou un distributeur de doses. Cette façon de procéder présente néanmoins le désavantage de nécessiter des machines beaucoup plus sophistiquées.

Selon une autre particularité de l'invention, l'éthylsuccinate d'érythromycine peut se présenter sous forme de microgranules de dimensions semblables à ceux de l'érythromycine base de telle sorte qu'un mélange homogène puisse facilement être réalisé avant la mise en gélules ou sachets. Cette façon de procéder rend possible le remplissage précis sur des machies plus simples et donc moins coûteuses.

Les microgranules d'érythromycine base se présentent sous forme de sphérules dont le diamètre est compris entre 0,05 mm et 3 mm, de préférence entre 0.1 mm et 2 mm.

Ils peuvent contenir des excipients tels que :
- les celluloses microcristallines, telles que les produits Avicel (F.M.C., U.S.A.)
- des charges telles que lactose, sucrose
- des amidons

- des méthylcelluloses, hydroxypropylcelluloses, carboxyméthylcelluloses,
- des polyvinylpyrrolidones
- des esters d'acides gras en C12 à C20 du saccharose, commercialisés sous le nom de sucroester (Gattefossé, France) ou sous le nom de Crodesta (Croda, U.K.)

Les microgranules d'érythromycine base peuvent être obtenus par des procédés connus, tels que le procédé d'extrusion - sphéronisation, ou le procédé du montage en turbine ou en lit fluidisé. excipients en présence d'un solvant, tel que l'eau ou un mélange hydroalcoolique, et à extruder la pâte ainsi obtenue au travers d'une filière.

A partir du produit extrudé, les microsphères sont obtenues après passage dans un appareil connu sous le nom de sphéroniseur. Les microgranules sont ensuite séchés et calibrés, au moyen de tamis appropriés au diamètre désiré.

Le procédé du montage en turbine ou lit fluidisé consiste au départ de microparticules à pistoler et/ou à saupoudrer l'érythromycine et les excipients jusqu'à obtention de microsphères de diamètre désiré. Les microgranules sont ensuite séchés et calibrés, au moyen de tamis appropriés au diamètre désiré.

Un mélange plastique convenant pour être granulé, extrudé et sphéronisé peut contenir les proportions pondérales suivantes :

| | | | |
|---|---|---|---|
| 40 % | à | 95 % | d'érythromycine base |
| 2 % | à | 15 % | Lactose |
| 0 % | à | 15 % | amidon de froment |
| 1 % | à | 20 % | Avicel CL 611 (cellulose microristalline de la firme FMC, USA) |
| 0 % | à | 5 % | Polyvinylpyrrolidone K30 |
| 0 % | à | 10 % | Sucroester WE15 (mouillant) |
| 0 % | à | 20 % | Avicel PH 101 (cellulose microcristalline de la firme FMC, USA) |
| 5 % | à | 35 % | eau distillée |

Ces microgranules sont ensuite recouverts d'une membrane entérosoluble par pulvérisation. Cette application peut s'effectuer par pulvérisation dans des turbines ou dans des lits fluidisés.

Parmi les polymères constituant la membrane entérosoluble, on peut citer en particulier les polymères d'acide métacrylique et d'ester méthylique d'acide méthacrylique (Eudragit L & S), les polymères d'acide polyméthacrylique et d'esters d'acide acrylique (L30D), les polymères d'hydroxypropylméthylcellulose phtalate, l'acétophtalate de cellulose, la carboxyméthyéthylcellulose.

Ces polymères peuvent être associés dans la membrane entérosoluble à au moins un adjuvant choisi parmi les suivants :
- plastifiants, tels que la triacétine, le dibutylphatlate, le dibutylsébaçate, les esters d'acide citrique, les polyéthylèneglycols, les polypropylèneglycols et la polyvinylpyrrolidone;
- pigments éventuellement colorés, tels que les oxydes de fer et l'oxyde de titane;
- mouillants, tels que les agents tensio-actifs des types Span et Tween, à savoir des esters partiels d'acides gras (acides laurique, palmitique, stéarique et oléique) et d'anhydrides d'hexitols dérivés du sorbitol contenant éventuellement des chaînes polyoxyéthyléniques, de préférence les agents tensio-actifs du type Tween, notamment le Tween 80, ainsi que les polyéthylèneglycols;
- lubrifiant, tel que le talc.

Outre le ou les polymères, la membrane entérosoluble contient, de préférence du talc à titre de lubrifiant, du dibutylphtalate à titre de plastifiant et du tween 80 comme mouillant.

Le poids de la membrane entérosoluble peut être de 5 à 40 %, de préférence de 10 à 30 % de celui des microgranules. Ces derniers peuvent contenir de l'érythromycine base à raison de 20 à 95 % en poids, de préférence de 65 à 85 % en poids.

La membrane entérosoluble peut contenir de 5 à 95 % et, de préférence, de 45 à 80 % de polymères ou mélange de polymères.

Selon une forme de réalisation exemplative de l'invention, la membrane microporeuse peut être obtenue au départ d'une dispersion aqueuse contenant en poids :

| | | | |
|---|---|---|---|
| 10 % | à | 70 % | Eudragit L30D (polymères) |
| 0 % | à | 10 % | talc (lubrifiant) |
| 0 % | à | 1 % | tween 80 |
| 25 % | à | 80 % | eau (solvant) |
| 1 % | à | 15 % | dibutylphtalate (plastifiant). |

Les microgranules d'éthylsuccinate d'érythromycine de la présente invention, destinés à libérer le principe

3

actif rapidement, peuvent être réalisés en mettant en oeuvre les mêmes techniques que celles utilisées pour les microgranules d'érythromycine base.

Une composition de mélange plastique covenant pour la granulation, l'extrusion et la sphéronisation peut contenir les proportions pondérales suivantes :

| | | | |
|---|---|---|---|
| 50 % | à | 90 % | éthylsuccinate d'érythromycine |
| 1 % | à | 10 % | Avicel CL 611 (cellulose microcristalline de la firme FMC, USA) |
| 1 % | à | 15 % | Avicel PH 101 (cellulose microcristalline de la firme FMC, USA) |
| 0 % | à | 10 % | amidon de froment |
| 0 % | à | 20 % | lactose |
| 0 % | à | 10 % | polyvinylpyrrolidone K30 |
| 10 % | à | 30 % | eau distillée |

La composition de la présente invention peut être obtenue en mélangeant les microgranules d'éthylsuccinate d'érythromycine avec les microgranules d'érythromycine base. Le mélange homogène ainsi obtenu peut être placé dans des capsules de gélatine dure (ou gélules).

Les microgranules peuvent être mélangés dans des proportions, exprimées en pourcentage d'érythromycine base, pouvant être comprises entre 20 % et 80 % et de préférence entre 30 % et 70 % d'érythromycine sous forme d'éthylsucciante par rapport à la quantité totale d'érythromycine présente dans la forme galénique.

L'ensemble des caractéristiques et avantages de l'invention sera mieux compris par l'homme de l'art en se référant à la description qui va suivre de modes de réalisation particuliers donnés à titre d'exemples non limitatifs de la nouvelle forme galénique de l'érythromycine, de son procédé de fabrication et de ses applications thérapeutiques, en particulier en relation avec des contrôles pharmacocinétiques utilisant cette nouvelle forme galénique.

<u>EXAMPLE N° 1</u>

I. A. Microgranules d'Erythromycine

I. A. 1. Fabrication des microgranules

On a utilisé les ingrédients suivants :

| | |
|---|---|
| Erythromycine base | 400 g |
| Lactose poudre | 50 g |
| Amidon de froment | 10 g |
| Avicel CL 611 | 33 g |
| Polyvinylpyrrolidone K90 | 8 g |
| | 501 g |

Après avoir introduit les poudres dans un mélangeur planétaire et les avoir granulés avec 130 g d'eau, la masse plastique a été extrudée au travers des trous d'un millimètre de diamètre d'une filière d'une extrudeuse (Fuji Paudal). Les cylindres obtenus ont été ensuite rendus sphériques par sphéronisation dans un appareil de type Caleva. Après séchage, pendant 12 heures, dans une étuve ventilée, portée à 50°C, la fraction de microgranules dont le diamètre est comprise entre 0,7 mm et 1,4 mm a été récupérée par tamisage au travers de tamis appropriés. On a ainsi obtenu 456g de microgranules (rendement : 91 %).

I. A. 2. Enrobage des microgranules

Sur 400 g de microgranules, dont la granulométrie est comprise entre 0,7 mm et 1,4 mm on a pistolé dans un appareil à lit fluidisé de marque Aeromatic type Strea I, 368 g de la dispersion suivante :

| | |
|---|---|
| Eudragit L30D | 495,3 g |
| Tween 80 | 1,0 g |
| Dibutylphtalate | 22,3 g |
| Eau | 481,4 g |
| | 1000,0 g |

Durée du pistolage : 40 minutes

Séchage dans étuve à 50°C pendant 12 heures.

I. B. Microgranules d'éthylsuccinate d'érythromycine
Pour la fabrication des microgranules, on a utilisé les quantités suivantes :

| | |
|---|---|
| Ethylsuccinate d'érythromycine | 4,00 kg |
| Avicel CL 611 | 0,32 kg |
| Amidon de froment | 0,10 kg |
| Lactose | 0,50 kg |
| Polyvinylpyrrolidone K90 | 0,08 kg |
| | 5,00 kg |

Eau pour granulation : 1,4 kg

En procédant comme pour la fabrication des microgranules d'érythromycine base (I.a.1.), on a obtenu 4,250 kg de microgranules dont le diamètre est comprise entre 0,7 et 1,4 mm (rendement 85 %).

I. C. Préparation de la forme pharmaceutique
Après contrôle du tire en érythromycine des microgranules, les quantités suivantes ont été mélangées pendant 15 minutes dans un mélangeur en "V" de marque Patterson Kelly.

| | |
|---|---|
| Microgranules d'érythromycine base (I.A.2.) | 350,0 g |
| Microgranules d'éthylsuccinate d'érythromycine | 485,6 g |
| | 835,6 g |

On a introduit dans des capsules de gélatine dure de taille "O" 379 mg du mélange pour obtenir des doses unitaires équivalentes à 250 mg d'érythromycine base.

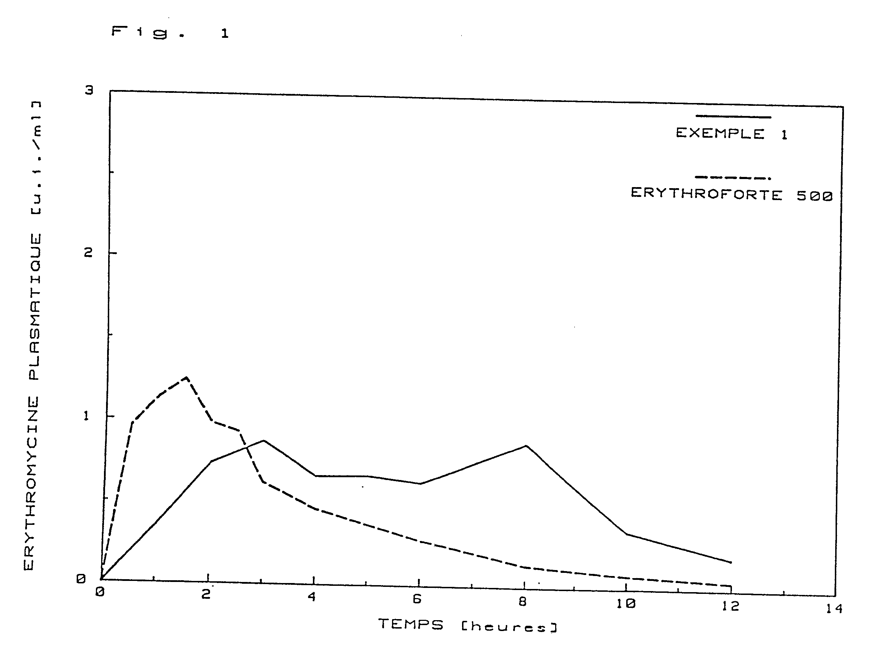

I. D. Etude pharmacocinétique
La forme galénque de l'exemple 1 à fait l'objet d'une étude comparative avec une suspension d'éthylsuccinate d'érythromycine disponible sur le marché : Erythroforte 500 (Abbot).
3 sujets volontaires sains ont reçu successivement à 2 semaines d'intervalle des doses équivalentes à 500 mg d'érythromycine base soit sous forme d'éthylsuccinate d'érythromycine soit sous forme de la forme galénique de l'exemple 1. La détermination de la cinétique des concentrations plasmatiques d'érythromycine a été effectuée au moyen de 8 à 10 prélèvements sanguins et a permis d'obtenir les courbes de la figure 1 ci-annexée.
Sur cette figure 1, la courbe en pointillé est relative à l'érythroforte 500 et la courbe en traite continu à la forme selon l'exemple 1.

Résultats :

I. D. 1. Biodisponibilité
D'après les valeurs des surfaces sous les courbes concentrations plasmatiques versus temps 0 - 12 heures (valeurs non extrapolées à l'infini), la biodisponibilité de la forme de référence sur le marché vaut 4,7 U.I./ml alors que celle de l'exemple 1 vaut 6,9 U.I./ml. Ceci permet de conclure que la biodisponibilité de la forme de l'exemple 1 est de 146 % par rapport à celle de la forme de référence.

TABLEAU 1

|  | Symbole | Unité | Erythroforte 500 | Exemple |
|---|---|---|---|---|
| Surface sous la courbe | AUC | U.I.h/ml | 4,7 | 6,9 |
| Concentrtion maximale | Cmax | U.I./ml | 1,3 | 1,2 |
| Temps de concentration maximale | Tmax | h | 1,2 | 5,0 |
| Temps durant lequel la concentration est supérieure à 75% de la concentration maximale | T75% | h | 1,1 | 2,7 |

Le tableau 1 permet de constater que l'allure des courbes plasmatiques des 2 produits testés est fondamentalement différente. En effet, bien que les concentrations maximales obtenues avec les 2 formes soient pratiquement identiques, le temps auquel elles apparaissent est extrêmement différent et beaucoup plus tardif pour la forme galénique de l'exemple 1. De même pour le temps durant lequel la concentration plasmatique d'érythromycine est supérieure à 75 % de la concentration maximale : 1,1 h versus 2,7 h, c'est-à-dire 245 %.

Ces paramètres permettent de démontrer que la forme galénique de l'exemple 1 possède des caractéristiques de libération soutenue d'érythromycine que ne possède pas la forme de référence.

EXEMPLE N°2

II. A. Migroganules d'érythromycine base

II. A. 1. Fabrication des microgranules
On a utilisé les quantités suivantes de produits exprimées en kilogrammes :

| | |
|---|---|
| Erythromycine base | 8,031 kg |
| Lactose | 0,705 kg |
| Amidon de froment | 0,200 kg |
| Avicel CL 611 | 0,604 kg |
| Polyvinylpyrrolidone K30 | 0,160 kg |
| Sucroester WE 15 | 0,300 kg |
| | 10,000 kg |
| Eau pour granulation | 2,200 kg |

Le mode de préparation était identique à celui de l'exemple 1. On a obtenu 9,374 kg de microgranules (rendement 94 %) dont le diamètre est compris entre 0,7 et 1,4 mm.

II.A.2. Enrobage des microgranules
On a opéré de la même manière que pour l'exemple 1.
Sur 700 g de microgranules (II.A.1.) on a pulvérisé 638,5 g de la dispersion aqueuse suivante :

| | |
|---|---|
| Talc | 42,6 g |
| Eudragit L30D | 433,3 g |
| Tween 80 | 1,0 g |
| Eau distillée | 499,4 g |
| Dibutylphtalate | 23,7 g |
| | 1000,0 g |

II.B. Microgranules d'éthylsuccinate d'érythromycine
En procédant de façon identique à l'exemple 1, les microgranules ont été obtenus au départ des quantités

6

suivantes :

| Ethylsuccinate d'érythromycine | 8,000 kg |
|---|---|
| Avicel CL 611 | 0,400 kg |
| Avicel pH 101 | 0,240 kg |
| Amidon de froment | 0,200 kg |
| Lactose | 1,000 kg |
| Polyvinylpyrrolidone K30 | 0,160 kg |
| | 10,000 kg |

Eau pour granulation : 0,225 kg

On a obtenu 8,392 kg de microgranules dont le diamètre est compris entre 0,7 mm et 1 mm (rendement 84 %).

II.C. Préparation de la forme pharmaceutique

En procédant comme dans l'exemple n°1, on a mélangé les quantités suivantes :

| Microgranules d'érythromycine base (II.A.2) | 500,0 g |
|---|---|
| Microgranules d'éthylsuccinate d'érythromycine (II.B) | 749,4 g |
| | 1249,4 g |

Les doses unitaires, dosées à 250 mg d'érythromycine base, ont été préparées en introduisant 367,13 mg du mélange de pellets dans des gélules de gélatine dure de taille "O".

II. D. Etude pharmacocinétique

II. D. 1. Administration unique

Une étude comparative de pharmacocinétique a été réalisée à la dose exprimée en érythromycine base de 500 mg. Cette étude a comparé la forme galénique de l'exemple n°2 à une forme actuellement sur le marché constituée d'éthylsuccinate d'érythromycine : ERYTHROFORTE de la firme Abbot.

Pour ce faire, dix-huit sujets volontaires sains ont reçu successivement chacune des 2 formes dans un ordre aléatoire à 2 semaines d'intervalle.

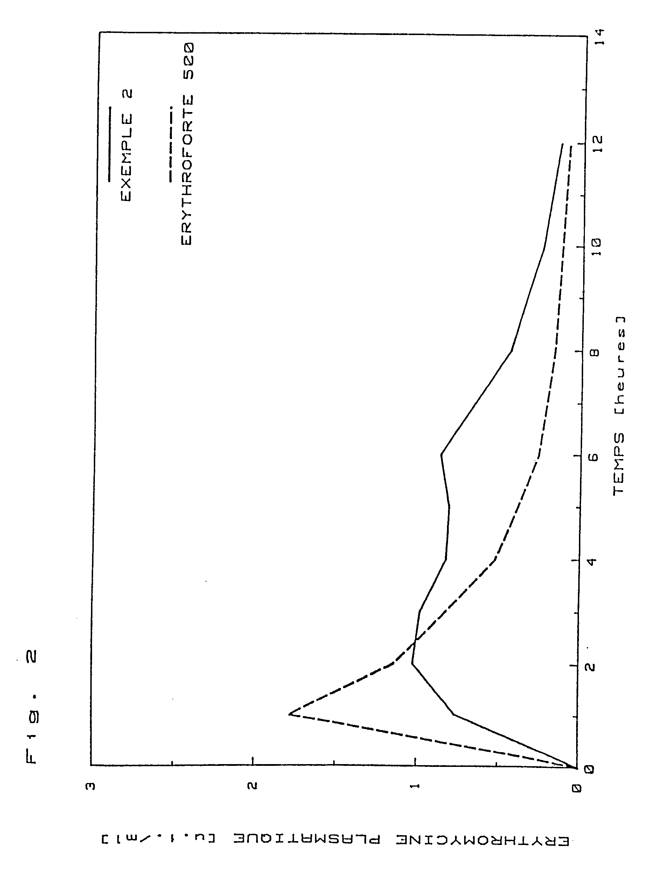

Les taux plasmatiques exprimés en Unités Internationales pour la mesure de l'évolution des concentrations sanguines ont été déterminés au moyen de 9 prélèvements sanguins. (voir fig. 2 ci-annexée).

Sur la figure 2, la courbe en pointillé est relative à l'érythroforte 500 [R] et la courbe en trait continu à la forme de l'exemple 2.

### TABLEAU 2

| | Symbole | Unité | Erythroforte 500 | Exemple 2 |
|---|---|---|---|---|
| Surface sous la courbe (0 - 12 h) | AUC | U.I.h/ml | 5,74 | 7,23 |
| Concentration maximale | Cmax | U.I./ml | 1,80 | 1,49 |
| Temps durant lequel la concentration est supérieure à 0.5 U.I./ml | T0,5 | h | 3,55 | 5,63 |

II. D. 1. A. Biodisponibilité

Les valeurs des surfaces sous les courbes, mesurées pour des temps compris entre 0 h et 12 h, permettent de constater que la forme de l'exemple 2 est au moins équivalente, du point de vue biodisponibilité, à celle de la forme de référence. En effet, la biodisponibilité de la forme de l'exemple 2 vaut 151 % de celle de la forme de référence.

II. D. 1. B. Allure des courbes

Les concentrations maximales obtenues avec la forme de l'exemple 2 sont inférieures et ne valent que 82 % de celles obtenues avec la forme de référence, alors que, simultanémentr, le temps durant lequel les concentrations plasmatiques mesurées avec la forme de l'exemple 2 sont supérieures à 0,5 U.I./ml est nettement supérieur à celui de la forme de référence (156,8 %).

Cette différence est hautement significative.

En conclusion : l'allure des courbes plasmatiques de la forme de l'exemple 2 est essentiellement différente de celle de la forme de référence et présente les caractéristiques d'un produit à libération prolongée d'érythromycine.

II.D.2. Administration répétée

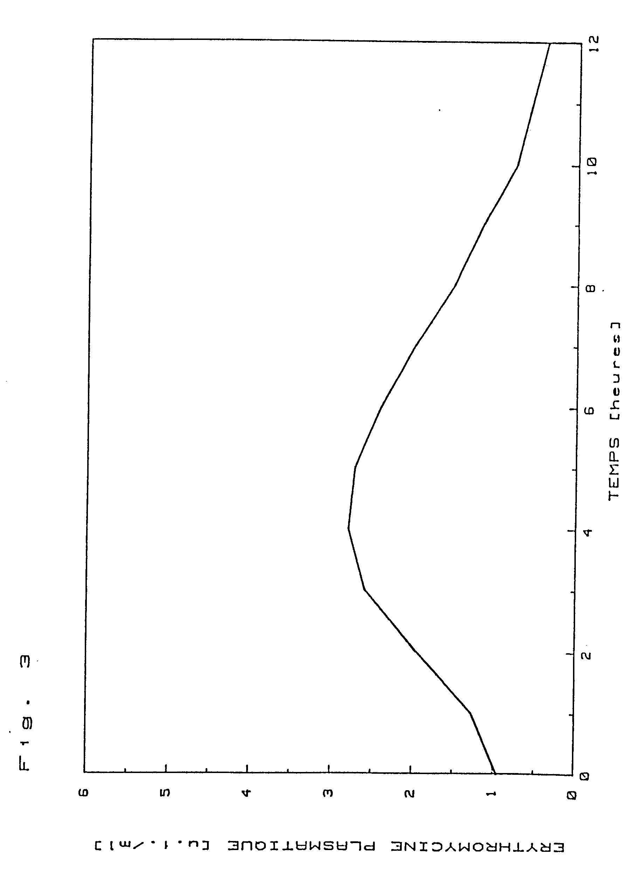

La forme galénique de l'exemple 2 a été administrée à 18 sujets volontaires sains à raison de 1 dose équivalant à 750 mg (3 gélules de 250 mg) toutes les 12 heures. Après la cinquième administration, les taux plasmatiques d'érythromycine, exprimés en Unités Internationales, sont mesurés pendant une durée de 12 heures. (fig. 3)

Au terme de cette étude, on peut donc conclure que :

La courbe moyenne des taux plasmatiques après la cinquième dose orale biquotitienne de la forme galénique de l'exemple 2 se situe pratiquement pendant 12 heures toujours audessus de 0,5 U.I./ml et pratiquement pendant 10 heures au-dessus de 1 U.I./ml. De plus, bien que les concentrations minimales soient élevées, la concentration maximale reste inférieure à 3 U.I./ml de plasma.

Ceci nous permet de dire que la forme galénique de la présente invention permet de maintenir des taux plasmatiques thérapeutiques efficaces entre 2 administrations successives espacées de 12 heures.

Il est entendu que l'homme de l'art pourra trouver d'autres avantages et variantes de l'invention, en particulier en ce qui concerne le procédé d'obtention des microgranules et de la membrane servant à contrôler la libération de la substance active, sans pour cela sortir du cadre et de la portée de la présente invention.

**Revendications**

1. Composition pharmaceutique à base d'érythromycine du type à libération prolongée, caractérisée en ce qu'elle comprend un mélange d'éthylsuccinate d'érythromycine et de microgranules d'érythromycine base entérosolubles.

2. Composition suivant la revendication 1, caractérisée en ce que l'éthylsuccinate d'érythromycine est sous la forme de microgranules.

3. Composition suivant l'une ou l'autre des revendications 1 et 2, caractérisée en ce que la quantité d'éthylsuccinate d'érythromycine par rapport à la quantité totale d'érythromycine base présente dans la composition, ces quantités étant exprimées en pourcentage d'érythromycine base, est de l'ordre de 20 à 80 %.

4. Composition suivant la revendication 3, caractérisée en ce que la quantité d'éthylsuccinate d'érythromycine est de l'ordre de 30 à 70 %.

5. Composition suivant l'une quelconque des revendications 1 à 3, caractérisée en ce qu'elle se présente sous la forme d'une dose unitaire ou posologique.

6, Composition suivant la revendication 5, caractérisée en ce qu'elle se présente sous forme de gélule , de comprimé ou de sachet.

7. Composition suivant l'une quelconque des revendications 1 à 4, caractérisée en ce qu'elle est présente dans un distributeur de doses.

8. Utilisation de la composition suivant l'une quelconque des revendications 1 à 7, pour la préparation d'un médicament pour le traitement des maladies microbiennes.

Fig. 1

Fig. 2

Fig. 3

TEMPS [heures]

ERYTHROMYCINE PLASMATIQUE [u.i./ml]

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | PHARMACOLOGY, vol. 29, no. 6, 1984, pages 305-311, S. Karger AG, Basel, CH; T.B. TJANDRAMAGA et al.: "Relative bioavailability of enteric coated pellets, stearate and ethylsuccinate formulations of erythromycin" * En entier * | 1-8 | A 61 K   31/71<br>A 61 K    9/54 |
| Y | US-A-4 250 166  (H. MAEKAWA) * Colonne 1, ligne 7 - colonne 2, ligne 14; colonne 6, lignes 20-25; colonne 10, ligne 13 - colonne 11, ligne 30 * | 1-8 | |
| A | EP-A-0 196 546  (ABBOTT LABORATORIES) * Page 1, lignes 1-7; page 2, ligne 34 - page 3, ligne 14; page 3, ligne 32 - page 4, ligne 2; exemple 1; revendications * | 1-8 | |
| A | FR-A-2 413 090  (ABBOTT LABORATORIES) * Page 1, lignes 1-15; page 2, lignes 6-28 * | | DOMAINES TECHNIQUES RECHERCHES (Int. Cl.5) |
| A | GB-A- 757 165  (ABBOTT LABORATORIES) * Page 1, lignes 22-69; page 1, ligne 83 - page 2, ligne 3; page 2, lignes 10-20 * | 1-8 | A 61 K |
| A | EP-A-0 129 382  (SHIONOGI & CO. LTD) * Page 1, lignes 3-11; page 3, ligne 18 - page 5, ligne 18; page 10, ligne 6 - page 13, lignes 6-22; exemples 1,2,12,16-19 * | 1-8 | |

Le présent rapport a été établi pour toutes les revendications

| Lieu de la recherche | Date d'achèvement de la recherche | Examinateur |
|---|---|---|
| LA HAYE | 30-08-1989 | MUELLNERS W. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P0402)